(19) Europäisches Patentamt
European Patent Office
Office européen des brevets

(11) **EP 4 390 393 A1**

(12) **EUROPEAN PATENT APPLICATION**

(43) Date of publication:
**26.06.2024 Bulletin 2024/26**

(21) Application number: **23219970.3**

(22) Date of filing: **22.12.2023**

(51) International Patent Classification (IPC):
***G01N 33/487*** (2006.01)

(52) Cooperative Patent Classification (CPC):
**G01N 33/48721**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 EP 22216619**

(71) Applicant: Imec VZW
**3001 Leuven (BE)**

(72) Inventors:
• **VERHULST, Anne**
**2235 Houtvenne (BE)**
• **VAN DORPE, Pol**
**3510 Spalbeek (BE)**
• **LAGAE, Liesbet**
**3000 Leuven (BE)**
• **HUYGHEBAERT, Cedric**
**3001 Heverlee (BE)**

(74) Representative: **Winger**
**Mouterij 16 bus 101**
**3190 Boortmeerbeek (BE)**

(54) **NANOPORE SENSING DEVICE COMPRISING MULTIPLE SENSING LAYERS**

(57)     In a first aspect, the present invention relates to a nanopore sensing device (10), comprising: (i) a nanopore (60) having a first orifice (61) and second orifice (62), and a length (L) running from the first (61) to the second (62) orifice; and (ii) one or more sensors (20, 30, 40) for sensing an electric feature in the nanopore (60); wherein the nanopore sensing device (10) comprises a plurality of sensing layers (22, 32, 42) arranged along the length (L), each sensing layer (22, 32, 42) being part of one of the sensors (20, 30, 40) and each adjacent pair of sensing layers (22, 32, 42) being separated by an isolating layer (51, 52, 53), and at least one of the sensors (20, 30, 40) is a field-effect transistor (20).

## FIG 2

## Description

## Technical field of the invention

The present invention relates to nanopore sensing devices and in particular to nanopore sensing devices based on a field-effect transistor.

## Background of the invention

Biosensors have been developed intensively during the last decades and have enabled a wide set of applications in life sciences. Among the different types of biosensors, nanopore sensors provide a unique advantage in that they offer single particle sensitivity, thereby allowing to characterize individual (bio)molecules-which was before essentially restricted to fluorescence spectroscopy and force spectroscopy. Accordingly, they have received considerable attention and a substantial number of device architectures therefor have been proposed and developed. Today, nanopore sensors are at the heart of state-of-the-art systems for DNA sequencing, and researchers are now setting their sights on proteomics, which is still a significantly more challenging task than DNA sequencing.

Whereas to date biological nanopores still outperform their solid-state counterparts for DNA-based applications, the latter offer many advantages for more complex single-particle sensing. This includes the ability to tailor the size, shape, and surface properties of the solid-state pore for optimal sensing; a formation process which is not stochastic and which results in devices which are physically and chemically more robust than their biological counterparts; and-last but not least-a more straightforward co-integration with electronic devices.

One promising candidate in this regard is that of a nanopore field-effect transistor (nanopore FET), in which the nanopore runs through or near the channel of the FET, such that the electrical conditions in the nanopore modulate the conductivity of the channel. As such, when a particle passes through the nanopore, it influences the source-drain current through the channel. Thus, a nanopore FET sensor can be formed, wherein translocation of a particle through the nanopore effectively affects the gating of the FET, which can in turn be used to detect and (partially) characterize the particle.

Moreover, Verhulst et al. (VERHULST, Anne S., et al. Boosting the Sensitivity of the Nanopore Field-Effect Transistor to Translocating Single Molecules. IEEE Sensors Journal, 2022, 22.6: 5732-5742.) showed that such a nanopore FET can have a unique boosting effect, thereby increasing the sensitivity up to 1000% and above.

However, one of the challenges in sensing non-DNA particles is that of the speed of said particles. While a plethora of molecular machines (typically enzymes) are available to reduce and control the speed with which DNA molecules pass through a nanopore; such velocity-reduction mechanisms are not readily available for other particles, including proteins. Even though nanopore FETs do have an edge with regard to detecting fast translocating particles-in part thanks to a larger detection bandwidth owing to the significantly larger electronic current used for sensing (as opposed to e.g. the ionic current used in a biological-nanopore detection system)-, there is another aspect to the speed-issue. Indeed, not only is the high (average) speed a challenge, even more problematic is that the speed is not controlled and is unknown. As such, a signal measured from a nanopore sensor may either come from a longer fast-moving particle or a shorter slow-moving one; with no way to distinguish between both.

There is thus still a need in the art for nanopore sensing devices which address at least some of the problems outlined above.

## Summary of the invention

It is an object of the present invention to provide good nanopore sensing devices which can allow to determine the speed and/or length of an analyte longitudinal to the nanopore. It is a further object of the present invention to provide good systems and methods associated therewith. This objective is accomplished by nanopore sensing devices, systems and methods according to the present invention.

It is an advantage of embodiments of the present invention that an analyte can be detected and/or (at least partially) characterized with good sensitivity. It is a further advantage of embodiments of the present invention that an electric fingerprint of the analyte can be determined.

It is an advantage of embodiments of the present invention that the FET sensor can be operated such as to benefit from a considerable 'boosting effect' in its sensitivity. It is yet a further advantage of embodiments of the present invention that the FET sensor can have a relatively high signal to noise ratio.

It is an advantage of embodiments of the present invention that the speed of an analyte longitudinal to the nanopore can be determined. It is a further advantage of embodiments of the present invention that the length of an analyte longitudinal to the nanopore can be determined. It is yet a further advantage of embodiments of the present invention that these can be determined with relatively high accuracy.

It is an advantage of embodiments of the present invention that the analyte can translocate through the nanopore at a substantially constant speed.

It is an advantage of embodiments of the present invention that the nanopore sensing device can have a relatively high output signal bandwidth. It is a further advantage of embodiments of the present invention that the high output signal bandwidth can be supported by a correspondingly large-bandwidth readout circuit.

It is an advantage of embodiments of the present invention that measures can be taken to reduce the complexity of manufacturing the nanopore sensing device. For example, it is an advantage of embodiments of the present invention that a FET sensor can be combined with a sensing layer and/or sensor which benefit the manufacturability of the sensing device as a whole. Similarly, it is an advantage of embodiments of the present invention that two or more sensing layers can share one or more contacts in common.

It is an advantage of embodiments of the present invention that the sensing layers may be closely about the nanopore, thereby maximizing their sensitivity to the nanopore's contents.

It is an advantage of embodiments of the present invention that it can provide a relatively high throughput of analyte analysis. It is a further advantage of embodiments of the present invention that this throughput can be increased by configuring multiple nanopore sensing devices in parallel. It is yet a further advantage of embodiments of the present invention that the nanopore sensing device can have a relatively small footprint, further increasing its ability to be parallelized.

It is an advantage of embodiments of the present invention that the nanopore sensing device can be employed for sensing a variety of analytes, including polypeptides and/or proteins. It is a further advantage of embodiments of the present invention that the nanopore sensing device can be relatively easily reconfigured between different applications without requiring significant changes.

As part of the present invention, the inventors came to the realization that the lack of speed and/or length determination of an analyte was hampering the potential of nanopore FETs. To meet this need, they invented a nanopore sensing device comprising multiple sensing layers and at least one FET sensor, thereby allowing to determine the speed and/or length of the analyte longitudinal to the nanopore through detecting the analyte at the different sensing layers as it is translocating through the nanopore, while nevertheless maintaining a highly sensitive reading of the analyte using the FET sensor.

In a first aspect, the present invention relates to a nanopore sensing device, comprising: (i) a nanopore having a first orifice and second orifice, and a length running from the first to the second orifice; and (ii) one or more sensors for sensing an electric feature in the nanopore; wherein the nanopore sensing device comprises a plurality of sensing layers arranged along the length, each sensing layer being part of one of the sensors and each adjacent pair of sensing layers being separated by an isolating layer, and at least one of the sensors is a field-effect transistor.

In a second aspect, the present invention relates to a system comprising the nanopore sensing device according to any embodiment of the first aspect and a microfluidic system coupled to the nanopore sensing device.

In a third aspect, the present invention relates to a method for sensing an analyte, comprising: (a) providing an analyte in a nanopore sensing device as defined in any embodiment of the first or second aspect; (b) translocating the analyte through the nanopore; and (c) sensing an electric feature in the nanopore as the analyte translocates therethrough.

In a fourth aspect, the present invention relates to a use of a nanopore sensing device as defined in any embodiment of the first aspect, for determining a speed and/or length of an analyte longitudinal to the nanopore.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

**Brief description of the drawings**

FIG 1 schematically depicts a nanopore sensing device in accordance with embodiments of the present invention, comprising two sensing layers: one being part of a FET and the other being a conductor layer.

FIG 2 schematically depicts a nanopore sensing device in accordance with embodiments of the present invention, comprising three sensing layers: two being part of a FET and the third being a conductor layer.

FIG 3 schematically depicts the signals one might obtain as an analyte translocates through a nanopore sensing device as in FIG 2.

FIG 4 schematically depicts a nanopore sensing device in accordance with embodiments of the present invention, comprising two sensing layers: one being part of an extended-gate FET and the other being a conductor layer.

FIG 5 schematically depicts a nanopore sensing device in accordance with embodiments of the present invention, comprising two sensing layers: both being part of a FET and sharing a common drain.

FIG 6 schematically depicts a nanopore sensing device in accordance with embodiments of the present invention, comprising three sensing layers: two being part of a FET and sharing a common source and drain, the third being a conductor layer.

In the different figures, the same reference signs refer to the same or analogous elements.

## Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

Reference will be made to transistors. These are devices having a first main terminal (e.g. a source or collector), a second main terminal (e.g. a drain or emitter) and a control terminal (e.g. a gate or base) for controlling the flow of electrical charges between the first and second main terminals.

The following terms are provided solely to aid in the understanding of the invention.

As used herein, and unless otherwise specified, a field-effect transistor (FET) is a type of transistor wherein an electric field controls the current flow between the source and drain through a semiconductor. Typically, a voltage applied at the third-i.e. gate-terminal creates and affects the size and shape of a conductive channel in the semiconductor, thereby controlling the flow of charge carriers between the source and drain. Structurally, a FET thus comprises a semiconductor as active (channel) layer. Functionally, a FET typically has a subthreshold swing at room temperature (e.g. 20 °C) of 150 mV/dec (millivolt per decade) or less. Note that while a FET typically comprises different regions and terminals, there need not be a strict junction between (all of) these. Indeed, there exist so-called 'junction-less FETs' wherein a source, channel and drain region are present in a single body (e.g. a single nanowire). Note that while a FET often comprises a source and drain region with identical doping type (e.g. both p-type or both n-type), this is a in general not necessary. Indeed, there e.g. exist so-called 'tunnel FETs' wherein the source and drain region have opposite doping type, and whereby the gate not only affects the size and shape of the conductive channel, but also locally (i.e. at the source-channel junction) sets the tunnel barrier height and width. Accordingly, it may be the source-channel junction which is dominant in determining the current in the subthreshold region. A tunnel FET can achieve subthreshold swings at room temperature far below 60mV/dec, essentially without lower limit. Note moreover that while a FET typically comprises one semiconductor as channel material, there need not be a limitation to one material. Indeed, there exist so-called 'heterostructure stacks' wherein the channel longitudinally (i.e. from source to drain) or laterally (i.e. parallel paths between source and drain) comprises stacked layers of semiconductor material. Such selections may be targeted at adding strain in the FET which affects the mobility, or at creating more favourable conduction through the channel by lowering the inherent tunnel barrier (e.g. for tunnel FETs).

As used herein, and unless otherwise specified, a nanopore field-effect transistor is a type of FET in which the role of the gate is effectively fulfilled by the nanopore. Accordingly, it is the electric potential profile in the nanopore which modulates the current between the source and drain. In turn the electric potential profile in the nanopore is affected by the content of the nanopore-including e.g. an analyte translocating therethrough-, and more specifically by the content's electric features (e.g. electric charge and/or electric dipole, making up the (local) electric field/electric potential variations inside or near the chemical entities in said content). As such, (changes in) the source-drain current can be used to detect and (at least partially) characterize an electric feature of the analyte, and by extension the analyte as a whole.

As used herein, and unless otherwise specified, an extended-gate field-effect transistor is a type of FET which has a 'main body' (e.g. with a source, drain, channel and gate) like a traditional FET, but wherein the gate is electrically (e.g. resistively) coupled to a (highly) conductor layer (e.g. a 2D conductor layer-such as graphene-or a metal-such as Pt, Ru, W, Al or Cu). The combination of a gate and conductor layer may then also be referred to as a 'long gate'. In embodiments, the gate may be electrically extended by means of the conductor layer as such. Alternatively, the gate may be electrically connected to the conductor layer by means of a further conductive material.

As used herein, and unless otherwise specified, sensing layers which have all their contacts in common yield a single output signal and are thus considered to be part of a single sensor. Conversely, sensing layers having at least one dedicated contact (e.g. one shared and one dedicated or both dedicated) typically yield two output signals and are thus considered to be part of two separate sensors.

In a first aspect, the present invention relates to a nanopore sensing device, comprising: (i) a nanopore having a first orifice and second orifice, and a length running from the first to the second orifice; and (ii) one or more sensors for sensing an electric feature in the nanopore; wherein the nanopore sensing device comprises a plurality of sensing layers arranged along the length, each sensing layer being part of one of the sensors and each adjacent pair of sensing layers being separated by an isolating layer, and at least one of the sensors is a field-effect transistor.

In embodiments, the field-effect transistor may have a subthreshold swing at room temperature of 150 mV/dec or less; preferably 120 mV/dec or less, more preferably 100 mV/dec or less, still more preferably 80 mV/dec or less, yet still more preferably 70 mV/dec or less, most preferably 60 mV/dec or less. In embodiments, the nanopore sensing device may be operated such (e.g. the cis and/or *trans* electrode may be biased such) that the field-effect transistor is operated in its subthreshold regime. Operating the field-effect transistor in its subthreshold regime allows it to have an exceedingly high sensitivity (e.g. due to a 'boosting effect'). This is typically generally useful, but particularly when the goal is not only to detect but also to characterize the analyte (cf. infra).

In embodiments, the field-effect transistor may be a nanopore field-effect transistor or an extended-gate field-effect transistor. In embodiments, the field-effect transistor may typically comprise a sensing layer which is a conductive or semiconductive part of said field-effect transistor. For a nanopore FET, the sensing layer may for example typically be the (semiconductive) channel of the FET. For an extended-gate FET, the sensing layer may for example typically be the conductor layer coupled to the gate (i.e. as part of the extended/long gate; and thus capacitively coupled to the channel). As such, the gating effect of the nanopore on the channel may be direct or indirect.

In the case of an extended-gate FET, the main body may preferably be located (very) close to (e.g. neighbouring)-but separated from (e.g. the latter does not go through it)-the nanopore. An advantage of an extended-gate FET may be that it can be easier to manufacture a FET with a gate extending to a conductor layer as sensing layer, compared to e.g. a nanopore field-effect transistor wherein the channel itself is the sensing layer.

Regardless of the nature of the field-effect transistor (e.g. nanopore FET or extended-gate FET), a difference in signal from the FET may preferably be substantially (e.g. exclusively) directly or indirectly (cf. supra) due to the gating effect of the nanopore (i.e. due to a change within the nanopore, such as the translocation of an analyte therethrough). In some embodiments using an extended-gate FET, the gate may be biased (i.e. not by the contents of the nanopore; but e.g. by an external voltage), so that a measured signal may be at least partially due to said bias (but a difference in signal remains substantially-preferably exclusively-due to the gating effect of the nanopore). Notwithstanding, in preferred embodiments (using a FET of any nature), the gate may not be biased. Accordingly, any measured signal may in preferred embodiments be substantially-preferably exclusively-due to the gating effect of nanopore.

In embodiments, the nanopore may run through-or at least near (sufficiently to have-directly or indirectly-a gating effect on the channel)-the sensing layer. In embodiments, the nanopore may be at least partially surrounded by the sensing layers. In preferred embodiments, the nanopore may be fully (i.e. 360°) surrounded by the sensing layer. In other words: the sensing layer may be at least partially-and preferably fully-about the nanopore. The more and closer the sensing layer surrounds the nanopore, the greater the gating effect of the nanopore-and therefore signal and signal-to-noise ratio-will typically advantageously be. In embodiments, the sensing layers and the isolating layers may form a stack and the nanopore may (completely) cross through said stack.

Typically, the sensing layers may be substantially parallel to one another. In embodiments, the nanopore may be oriented at an angle (different from 0; e.g. the length may be oriented at said angle) to the sensing layers. The angle may for example be from 30 to 150°, preferably from 60 to 120°, more preferably from 85 to 95°, most preferably 90°. Thus, if the sensing layers are considered to be oriented horizontally (i.e. they are substantially parallel to the horizontal plane), the nanopore may preferably be substantially vertical (i.e. the length may extend vertically). Accordingly, the sensing layers (and isolating layer(s)) may preferably be stacked substantially vertically. In embodiments, the source and a region may define a source-drain axis and the nanopore may be oriented at an angle (different from 0; e.g. the length may be oriented at said angle) to the source-drain axis. The angle may again for example be from 30 to 150°, preferably from 60 to 120°, more preferably from 85 to 95°, most preferably 90°. Thus, the source-drain axis-which will typically be parallel to the sensing layers (especially to the one sensing layer forming the FET sensor with said source and drain)-may in particular be perpendicular to the nanopore. If the nanopore is considered to be vertical, such a FET may then be referred to as a 'horizontal FET' (e.g. as opposed to a 'vertical FET', where the source-drain axis is parallel to nanopore). Horizontal FETs (and horizontal sensing layers/sensors in general) are advantageous in that this orientation typically takes up the least amount of spaces along the nanopore length, thereby allowing to efficiently stack the plurality of sensing layers (and sensors) without requiring an excessively long nanopore.

In general, the sensing layers may be semiconductor layers and/or conductor layers. For example, each sensing layer may independently be a semiconductor layer or a conductor layer.

Typically, at least the sensing layer comprised in the field-effect transistor will be a semiconductor layer. In embodiments, one or more further sensing layers may be semiconductor layers. In embodiments, each semiconductor layer may comprise a semiconductor material independently selected from group IV (e.g. Si, Ge or SiGe), III-V (e.g. GaAs, InGaAs or InP), 2D (e.g. a transition metal dichalcogenide, graphene or hexagonal boron nitride) semiconductors or semiconducting carbon nanotubes (CNTs). In embodiments, the semiconductor layer may comprise more than one semiconductor material (e.g. a stack of semiconductor materials, like a heterostructure semiconductor stack). As such, the semiconductor channel material may be different from the source material and/or from the drain material (e.g. forming a heterojunction at the source-channel and/or channel-drain junction).

In embodiments, at least one of the sensing layers may be a conductor layer. In embodiments, each conductor layer may comprise a conducting material independently selected from 2D conductors (e.g. graphene), metals (e.g. Pt, Ru, W, Al or Cu) or metallic CNTs. In embodiments, the conductor layer may be part of a conductive sensor (e.g. comprising the conductive sensing layer and two contacts to said sensing layer) or an extended-gate field-effect transistor (cf. supra). Sensors comprising a conductor layer as sensing layer typically have in common that they are easier to fabricate than those based on semiconductor layers and may in some instances thus be chosen for that reason. Similar to a FET, in a nanopore conductive sensor, the resistance/conductance through the active layer (i.e. here the conductive sensing layer) is modulated by the contents of the nanopore. However, this effect is typically much (e.g. several orders of magnitude) smaller in a conductor layer than it can be in a FET, so that conductive sensors are typically considerably less sensitive than FET sensors. Hence, it is within the present invention necessary that at least one of the sensors is a FET, so that at least one highly sensitive sensor is present (and thus the signal thereof can e.g. be used to characterize the analyte). Notwithstanding, while typically insufficient for e.g. characterization of the analyte, conductive sensors may be adequate to detect the presence of the analyte and thus allow to use this information (e.g. in combination with that of the FET sensor) to determine for instance the speed and/or length of the analyte.

In embodiments, the isolating layer may be an insulating (i.e. non-conducting) layer. In embodiments, the isolating layer may comprise a dielectric, such as $SiO_2$.

In embodiments, the isolating layers may have a thickness of between 2 nm and 100 nm; preferably between 3 nm and 50 nm, more preferably between 4 nm and 25 nm, still more preferably between 5 nm and 10 nm.

Depending on the nature of the sensing layer (e.g. in view of their stability when in contact with the nanopore's contents), there may be a need and/or desire to further provide a thin protective layer between the sensing layer and the (inside of) the nanopore. This is for example typically the case for Si, while e.g. Pt is typically stable regardless. In embodiments, the protective layer may for example be an oxide (e.g. $SiO_2$ or $RuO_2$) or nitride (e.g. SiN) of (one of) the sensing layers. The protective layer is preferably sufficiently thin so as to not compromise the sensitivity of the sensing layer to the nanopore's contents too much; for example, the protective layer may have an 'effective oxide thickness' (i.e. relative to $SiO_2$) of 3 nm or below, preferably 2 nm or below, more preferably 1 nm or below. Such a protective layer may also be beneficial to make the nanopore more uniform (e.g. particularly in nature/surface charge, but also in width); cf. infra. Accordingly, not only one or more sensing layers but a substantial part (e.g. at least 50%, preferably at least 70%, yet more preferably at least 90%) of the section between the uppermost sensing layer and the lowermost sensing layer section may be lined with the protective layer.

In the case of the extended gate, that oxide (e.g. for Ru) could be Ru-oxide, and some materials do not need an oxide (Pt) but will be stable in a metal-fluid connection.

In embodiments, the nanopore may have a length of from 1 nm to 1 $\mu$m, preferably from 2 nm to 200 nm, more preferably from 5 nm to 100 nm, most preferably from 10 nm to 50 nm.

In embodiments, the nanopore may have a width of between 1 and 200 nm; preferably between 3 and 100 nm, most preferably between 5 nm and 30 nm. The width may typically be the widest dimension of a cross-section through the nanopore. In embodiments, the width may be the maximal width or the average width. In embodiments wherein the nanopore has a circular cross-section (i.e. the nanopore is cylindrical), the width may be the diameter of the nanopore. That said, the nanopore is not typically limited by its cross-section, and the cross-section may for example be circular, oval, triangular, rectangular, square, pentagonal, hexagonal, etc. Notwithstanding, a circular cross-section may in some instances nevertheless be preferred, as it may allow for the highest sensitivity for the sensing layers (particularly if the analyte is in first approximation rather spherical or cylindrical).

The nanopore may typically have a section defined between an uppermost sensing layer (e.g. a top thereof) and a lowermost sensing layer (e.g. a bottom thereof). In embodiments, said section may have a substantially constant width. In embodiments, said section may have substantially uniform sidewalls. In preferred embodiments, said section may have both a substantially constant width and substantially uniform sidewalls. A nanopore which is substantially uniform (particularly in width and nature) across the sensing region (i.e. across the section between the uppermost and lowermost sensing layer) is advantageous in that it promotes the analyte to have a constant speed throughout said sensing region. In embodiments, the width may be substantially constant if any local width differs from the average width by less than 20%, preferably less than 15%, yet more preferably less than 10%. In embodiments, the sidewalls may be substantially uniform may be substantially uniform if the sidewalls have a substantially uniform surface charge. In embodiments, the surface charge may be substantially uniform if any local surface charge differs from the average surface charge by less than 15%, preferably less than 10%, yet more preferably less than 5%. For example, the sidewalls may be made up of substantially the same material.

In alternative embodiments, the nanopore may have a smaller width at the sensing layers. Such a nanopore may advantageously be particularly suited for sensing longer analytes, as it reduces the total resistance in the nanopore, which has a positive impact on the overall signal-to-noise ratio of the nanopore sensing device.

In embodiments, the nanopore may have an above average (i.e. wider) width above the uppermost sensing layer and/or below the lowermost sensing layer. This can advantageously aid in facilitating entry into and evacuation out of the nanopore for the analyte.

In embodiments, at least two of the sensing layers may have one or two contacts in common. In embodiments, the one or two common contacts may be a common source and/or a common drain. Sharing contacts may advantageously improve the ease with which the sensors can be fabricated. Note that in the case where sensing layers share all their contacts, they will also output a single signal. Nevertheless, while it adds some complication, it is typically possible (e.g. during post-processing of the signal) to deconvolute said signal into signals for the individual sensing layers, or at least to derive the relevant sensing events at the different sensing layers from the convoluted signal. This situation could therefore be regarded as a kind of trade-off between ease of manufacture and ease of signal processing.

In embodiments, the nanopore sensing device may further comprise a readout circuitry. In embodiments, the readout circuitry may comprise an amplifier. A readout circuitry may for example typically comprise one or more further field-effect transistors. Notwithstanding, it will be understood that such FETs are distinct from- and not to be confounded with-the at least one sensor which is a FET as defined in the first aspect. Indeed, such further FETs do not comprise a sensing layer (sensitive to an electric feature in the nanopore) arranged along the length of the nanopore.

In embodiments, the readout circuit may have a bandwidth of at least 10 kHz; preferably at least 100 kHz; more preferably at least 500 kHz, still more preferably at least 1 MHz. Nanopore sensing device in accordance with the present invention can advantageously typically have a relatively high readout bandwidth. In embodiments, the sensors may output one or more signals at the aforementioned bandwidth (combined or individually).

In embodiments, the field-effect transistor may have a signal-to-noise ratio of at least 0 dB up to at least 100 kHz; preferably at least 1 MHz, more preferably at least 10 MHz. The field-effect transistor in nanopore sensing device in accordance with the present invention can typically advantageously have a relatively high sensitivity (and thus signal-to-noise ratio). This may for example be at least in part thanks to the 'boosting' effect achieved when operating the field-effect transistor in its subthreshold regime.

In embodiments, any feature of any embodiment of the first aspect may independently be as correspondingly described for any embodiment of any of the other aspects.

In a second aspect, the present invention relates to a system comprising the nanopore sensing device according to any embodiment of the first aspect and a microfluidic system coupled to the nanopore sensing device.

The microfluidic system may for example comprise a plurality of microfluidic channels. In embodiments, the microfluidic system may further comprise a (microfluidic) pump for generating and/or controlling a fluid flow through the microfluidic system.

In embodiments, the system may further comprise: (iii) a cis reservoir and a *trans* reservoir; and (iv) a cis electrode and a *trans* electrode, for generating a potential difference for translocating an analyte through the nanopore.

In embodiments, the system may further comprise a control unit. The control unit may for example be configured for controlling the nanopore sensing device and/or the microfluidic system. In embodiments, the control unit may be configured for performing step c of the method according to the third aspect (cf. infra).

In embodiments, any feature of any embodiment of the second aspect may independently be as correspondingly described for any embodiment of any of the other aspects.

In a third aspect, the present invention relates to a method for sensing an analyte, comprising: (a) providing an analyte in a nanopore sensing device as defined in any embodiment of the first or second aspect; (b) translocating the analyte through the nanopore; and (c) sensing an electric feature in the nanopore as the analyte translocates therethrough.

In embodiments, providing the analyte in the nanopore sensing device may comprise providing a dispersion (e.g. a solution, colloid or suspension) of the analyte in a liquid (e.g. water, possibly with a buffer added). In embodiments, providing the analyte may comprise providing said dispersion above or below the nanopore. In embodiments, providing the analyte may comprise providing said dispersion in the cis reservoir or in the *trans* reservoir.

The invention is typically not particularly limited by the type of analyte, provided it can be translocated through the nanopore. In embodiments, the analyte may thus generally be any (single) molecule or (single) particle, such as in particular a charged molecule, charged particle, biomolecule or bioparticle. Notwithstanding, the analyte may in preferred embodiments be a polypeptide, a protein (e.g. comprising one or more polypeptides) or a nucleic acid (e.g. a deoxyribonucleic acid-DNA; or a ribonucleic acid-RNA). Such analytes may be preferred in that the interest and usefulness for single molecule/particle detection and/or characterization is at this moment especially great for these types of bioanalytes, while e.g. compared to other techniques those of the present invention may be particularly suited therefor.

In embodiments, translocating the analyte through the nanopore may comprise generating a potential difference between the cis and *trans* electrode. It will be clear that movement-and thus translocation through the nanopore-of an analyte under a potential difference will typically depend on its charge and the fluid convection flow which is generated. For instance, if a more positive voltage is applied to the cis electrode than to the *trans* electrode, a negatively charged analyte will tend to move from the *trans* to the cis reservoir; a positively charged analyte will tend to move from the *cis* to the *trans* reservoir; and a neutral analyte will tend to follow the fluid convection flow (induced by the movement of e.g. salt ions, and which will thus depend on these).

In embodiments, step c may comprising sensing the electric feature at a plurality (e.g. each) of the sensing layers.

In embodiments, sensing an electric feature in the nanopore may comprise measuring a current through the sensor. In general, the sensing layer and sensor are typically such that at least one electric characteristic thereof is modulated by the contents of the nanopore, which can in turn be registered as a change in signal of (e.g. a change in current through) the sensor. For example, in the case of a nanopore FET or a sensor based on a conductor layer, the resistance/conductance of the sensing layer as such may be influenced by contents of the nanopore. Conversely, in the case of an extended-gate FET, the contents of the nanopore typically affects the voltage of the sensing layer-and thus the extended gate-, which will in turn change the resistance/conductance of the FET's channel region.

In embodiments, the method may further comprise: (d) analysing the electric feature(s) sensed in step c to determine therefrom a speed and/or length of the analyte longitudinal to the nanopore. In embodiments, step d may comprise deriving from said electrostatic potential data a plurality of event times (e.g. arrival and/or departure times) of the analyte corresponding to the plurality of sensing layers. Since the distance between sensing layers is typically known, these can then be used to calculate a speed and/or a length of the analyte. This can for instance be done based on equations like:

$$v_a = \frac{\Delta x_{1 \to 2}}{\Delta t_{1 \to 2}} \text{ and/or } l_a = v_a \times \Delta t_a;$$

wherein $v_a$ is the speed (i.e. velocity) of the analyte longitudinal to the nanopore, $\Delta x_{1 \to 2}$ is the distance (e.g. height) between two sensing layers 1 and 2 and $\Delta t_{1 \to 2}$ is the time (i.e. duration) between registering an event of the analyte at the sensing layers 1 and 2 (e.g. the difference in arrival times, or in departure times), $l_a$ is the length of the analyte longitudinal to the nanopore, and $\Delta t_a$ is the signal duration over which the analyte was detected at a sensing layer (e.g. the difference between arrival and departure times for sensing layer 1, or for sensing layer 2, or the average of these). Obviously, for more than two sensing layers, these values can be calculated multiple times (e.g. $1 \to 2$, $2 \to 3$, $1 \to 3$, etc.) and thus increases the robustness of the measurement. For example, this may be used to check whether the speed through the nanopore (or more specifically: between the sensing layers) is constant or-assuming a constant speed-to increase the accuracy of the determined value (by statistically reducing the noise through the multiple sampling).

In embodiments, the method may further comprise: (e) determining an electric fingerprint of the analyte. In embodiments, step e may be performed separately or together with step d. In embodiments, determining an electric fingerprint of the analyte may comprise determining a plurality of the electric features sensed as the analyte translocates through the nanopore. The fingerprint may be advantageously used to characterize the analyte. This could for example be done by providing (e.g. experimentally or through simulations) a number of different analyte fingerprints and comparing the determined analyte fingerprint thereto, to look for any (full or partial) match.

In embodiments, any feature of any embodiment of the third aspect may independently be as correspondingly described for any embodiment of any of the other aspects.

In a fourth aspect, the present invention relates to a use of a nanopore sensing device as defined in any embodiment of the first aspect, for determining a speed and/or length of an analyte longitudinal to the nanopore.

In embodiments, any feature of any embodiment of the fourth aspect may independently be as correspondingly described for any embodiment of any of the other aspects.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

**Example 1:** Nanopore sensing devices comprising a nanopore FET

***Example 1A:*** *Nanopore sensing devices comprising two sensing layers*

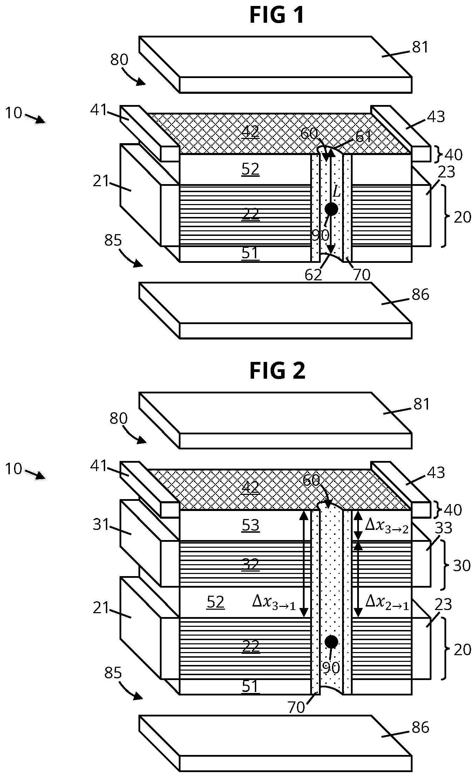

We now refer to FIG 1, illustrating a nanopore sensing device (10) comprising two sensing layers (22, 42). Above the nanopore sensing device (10) is a cis reservoir (80) and a cis electrode (81), while below it is a *trans* reservoir (85) and a *trans* electrode (86).

The nanopore sensing device (10) itself comprises a first isolating layer (51), a first sensing layer (22), a second isolating layer (52) and a second sensing layer (42) stacked on top of one another, with a nanopore (60) crossing through the stack and opening up to the cis reservoir (80) and *trans* reservoir (85) on either side. An oxide (70) lines the nanopore (60) from the first isolating layer (51) to the second isolating layer (52) (i.e. not the second sensing layer (42)).

The first sensing layer (22) is a semi-conductor layer and is part of a first sensor (20), namely a nanopore FET. The nanopore FET (20) comprises a source (21) and drain (23), the first sensing layer (22) as (or at least comprising) the channel region, and is gated by (the contents of) the nanopore (60). The nanopore FET (20) may for instance be a Si nanopore FET.

The second sensing layer (42) is a conductor layer and is part of a second sensor (40). The second sensor comprises said conductive sensing layer (42) and a first (41) and second (43) contact to the latter. The resistance of the conductive sensing layer (42) is modulated by (the contents of) the nanopore (60). The conductive sensing layer (42) may for instance be graphene, a (thin) metal (e.g. Pt or Ru) or a metallic CNT.

By operating the *cis* (81) and *trans* (86) electrodes, a potential difference across the *cis* (80) and *trans* (85) reservoirs-and thus across the nanopore (60)-is generated, which can be used to translocate an analyte (90) through the nanopore (60) (cf. supra).

***Example 1B:*** *Nanopore sensing devices comprising three sensing layers*

We now refer to FIG 2, illustrating a nanopore sensing device (10) which is generally similar to that of FIG 1 but comprises three sensing layers (22, 32, 42).

The nanopore sensing device (10) of FIG 2 comprises a first isolating layer (51), a first sensing layer (22), a second isolating layer (52), a second sensing layer (32), a third isolating layer (53) and a third sensing layer (42) stacked on top of one another, with a nanopore (60) crossing through the stack and opening up to the cis reservoir (80) and *trans* reservoir (85) on either side. An oxide (70) again lines the nanopore (60) from the first isolating layer (51) to the third isolating layer (i.e. not including third sensing layer).

The first sensing layer (22) is as described for the first sensing layer of FIG 1.

The second sensing layer (32) is also a semiconductor layer and is part of a second sensor (30), which is also a nanopore FET. This nanopore FET (30) comprises a further source (31) and drain (33), the second sensing layer (32) as (or at least comprising) its channel region and is also gated by (the contents of) the nanopore (60). The second nanopore FET (30) may for instance be a backend-of-the-line (BEOL) nanopore FET.

The third sensing layer (42) is as described for the second sensing layer of FIG 1.

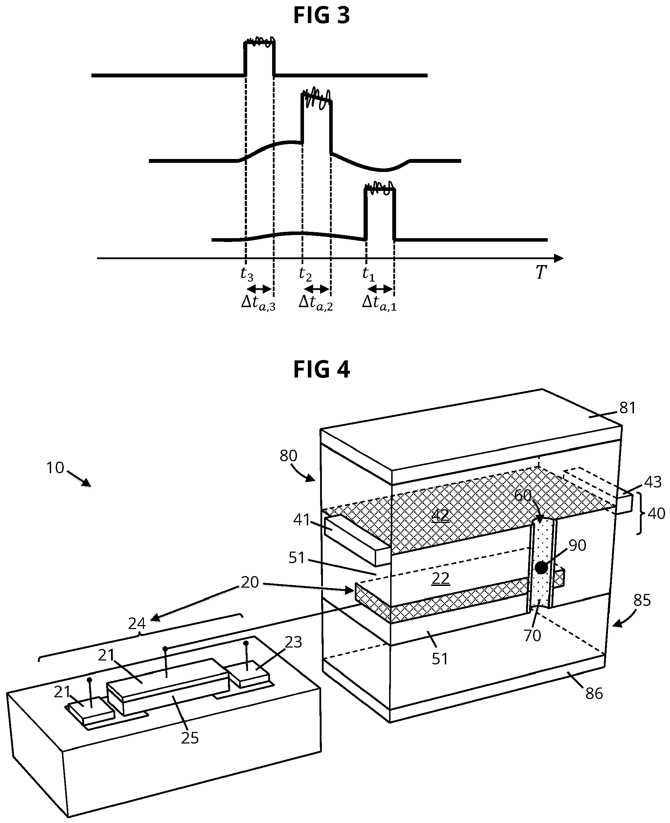

We now refer to FIG 3, illustrating the signals over time ($T$) from the third (top), second (middle) and first (bottom) sensor one might obtain as an analyte (90) translocates through the nanopore (60) sensing device (10) as in FIG 2 (here from the third to the first sensor). Generally each signal comprises a superposition of a background signal (here depicted as a deviation from the baseline), a main electric feature (depicted as a box pulse on top of the background signal) and a fine local variations in the dominant electric feature (depicted as a squiggle over the box). The magnitude (both in absolute terms and relative to the other features) and other characteristics of these typically depend on each individual sensor. For example, the pulse height of the main feature (relative to the background) is not constant and may e.g. be lower for a conductive sensor than for a FET sensor. Likewise, some sensors may be largely sensitive only to local changes in electric field (i.e. near the sensor), while for others e.g. the background signal may be significantly determined by the contents of the nanopore as a whole.

As can be see, the three sensing layers allow to define three arrival (or departure) times $t_3$, $t_2$ and $t_1$; three interlayer distances $\Delta x_{3 \to 2}$, $\Delta x_{2 \to 1}$ and $\Delta x_{3 \to 1}$ (see FIG 2); three signal durations $\Delta t_{a,3}$, $\Delta t_{a,2}$ and $\Delta t_{a,1}$; etc. And thus also to calculate three instances of the speed $v_{a,3 \to 2}$, $v_{a,2 \to 1}$ and $v_{a,3 \to 1}$; and three instances of the length $l_{a,3 \to 2}$, $l_{a,2 \to 1}$ and $l_{a,3 \to 1}$. By contrast, in the case of only two sensing layers, only one of each of these can be determined. Accordingly, using more than two sensing layers (e.g. three, four, etc.) allows to increase the robustness of the measurements/calculations, e.g. by statistically increasing their accuracy.

**Example 2:** Nanopore sensing devices comprising an extended-gate FET

We now refer to FIG 4, illustrating a nanopore sensing device (10) which is generally similar to that of FIG 1 but comprises one sensing layer (22) which is part of an extended-gate FET (20).

The nanopore sensing device (10) of FIG 4 comprises a first isolating layer (51), a first sensing layer (22), a second isolating layer (52) and a second sensing layer (42) stacked on top of one another, with a nanopore (60) crossing through the stack and opening up to the cis reservoir (80) and *trans* reservoir (85) on either side. An oxide (70) again lines the nanopore (60) from the first isolating layer (51) to the second isolating (i.e. not the second sensing layer (42)).

The first sensing layer (22) is a conductor layer but is part of an extended-gate FET as first sensor (20). The extended-gate FET (20) comprises a main body (24) comprising a source (21), a drain (23), a channel (25) and a gate (26). The gate (26) is however electrically coupled-and thereby extended ('long gate')-to the conductive first sensing layer (22). The FET's main body (24) may for instance be a Si FET, while the conductive first sensing layer (22) may for instance be graphene, a (thin) metal (e.g. Pt or Ru) or metallic CNT.

The second sensing layer (42) is as described for the second sensing layer (42) of FIG 1.

**Example 3:** Nanopore sensing devices wherein (some) sensor layers share a common contact

***Example 3A:*** *Nanopore sensing devices with one common contact*

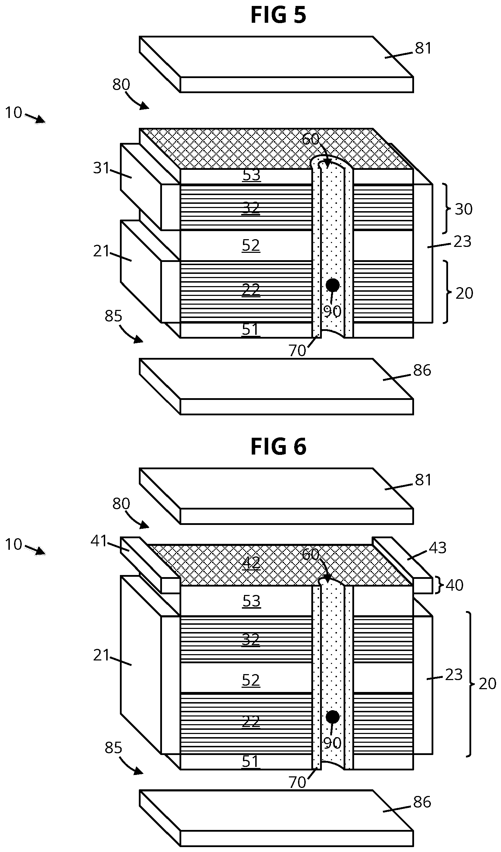

We now refer to FIG 5, illustrating a nanopore sensing device (10) which is generally similar to that of FIG 2 but comprises two sensing layers (22, 32) sharing a common drain (23).

The nanopore sensing device (10) of FIG 5 comprises a first isolating layer (51), a first sensing layer (22), a second isolating layer (52), a second sensing layer (42) and a third isolating layer (53) stacked on top of one another, with a nanopore (60) crossing through the stack and opening up to the cis reservoir (80) and *trans* reservoir (85) on either side. An oxide (70) again lines the nanopore (60) from the first isolating layer (51) to the third isolating (53-.

The first (22) and second (32) sensing layers are generally as described for the first and second sensing layers of FIG 2, but instead of each having their own dedicated drain they both share one common drain (23). Notwithstanding, since the first (22) and second (32) sensing layers still have individual source contacts (21, 31), they are nevertheless considered to be part of two separate sensors (20, 30).

*Example 3B: Nanopore sensing devices with two common contacts*

We now refer to FIG 6, illustrating a nanopore sensing device (10) which is generally similar to that of FIG 5 but comprises two sensing layers (22, 32) sharing both a common source (21) and common drain (23).

The nanopore (60) sensing device (10) of FIG 6 comprises a first isolating layer (51), a first sensing layer (22), a second isolating layer (52), a second sensing layer (32), a third isolating layer (53) and a third sensing layer (42) stacked on top of one another, with a nanopore (60) crossing through the stack and opening up to the cis reservoir (80) and *trans* reservoir (85) on either side. An oxide (70) again lines the nanopore (60) from the first isolating layer (51) to the third isolating (53) (i.e. not the third sensing layer).

The first (22) and second (32)sensing layers generally are as described for the first and second sensing layers of FIG 5, but now both sharing one common source (21) and one common drain (23). Since the first (22) and second (32) sensing layers now share all their contacts (21, 23), they are considered to be part of a single sensor (20).

The third sensing layer is as described for the third sensing layer of FIG 2.

It is to be understood that although preferred embodiments, specific constructions, configurations and materials have been discussed herein in order to illustrate the present invention. It will be apparent to those skilled in the art that various changes or modifications in form and detail may be made without departing from the scope of the invention as defined in the appended claims.

**Claims**

1. A nanopore sensing device (10), comprising:

    i. a nanopore (60) having a first orifice (61) and second orifice (62), and a length (L) running from the first (61) to the second (62) orifice; and
    ii. one or more sensors (20, 30, 40) for sensing an electric feature in the nanopore (60); wherein

        - the nanopore sensing device (10) comprises a plurality of sensing layers (22, 23, 24) arranged along the length (L),

            each sensing layer (22, 32, 42) being part of one of the sensors (20, 30, 40) and
            each adjacent pair of sensing layers (22, 32, 42) being separated by an isolating layer (51, 52, 53), and

    - at least one of the sensors (20, 30, 40) is a field-effect transistor (20).

2. The nanopore sensing device (10) according to claim 1, wherein the field-effect transistor (20) has a subthreshold swing at room temperature of 150 mV/dec or less; preferably 120 mV/dec or less, more preferably 100 mV/dec or less, still more preferably 80 mV/dec or less, yet still more preferably 70 mV/dec or less, most preferably 60 mV/dec or less.

3. The nanopore sensing device (10) according to any of the previous claims, wherein the field-effect transistor (20) is a nanopore field-effect transistor or an extended-gate field-effect transistor.

4. The nanopore sensing device (10) according to any of the previous claims, wherein at least two of the sensing layers (22, 32, 42) have one or two contacts in common (21, 23).

5. The nanopore sensing device (10) according to any of the previous claims, wherein at least one of the sensing layers (22, 32, 42) is a conductor layer.

6. The nanopore sensing device (10) according to any of the previous claims, wherein the isolating layers (51, 52, 53) have a thickness of between 2 nm and 100 nm; preferably between 3 nm and 50 nm, more preferably between 4 nm and 25 nm, still more preferably between 5 nm and 10 nm.

7. The nanopore sensing device (10) according to any of the previous claims, wherein the nanopore (60) has a width of between 1 and 200 nm; preferably between 3 and 100 nm, more preferably between 5 nm and 30 nm.

8. The nanopore sensing device (10) according to any of the previous claims, wherein the nanopore (60) has a section defined between an uppermost sensing layer (32, 42) and a lowermost sensing layer (22), wherein said section has a substantially constant width and substantially uniform sidewalls.

9. The nanopore sensing device (10) according to any of the previous claims, wherein the nanopore (60) has an above average width above the uppermost sensing layer (32, 42) and/or below the lowermost sensing layer (22).

10. The nanopore sensing device (10) according to any of the previous claims, further comprising a readout circuit having a bandwidth of at least 10 kHz; preferably at least 100 kHz; more preferably at least 500 kHz, still more preferably at least 1 MHz.

11. The nanopore sensing device (10) according to any of the previous claims, wherein the field-effect transistor (20) has a signal-to-noise ratio of at least 0 dB up to at least 100 kHz; preferably at least 1 MHz, more preferably at least 10 MHz.

**12.** A system comprising the nanopore sensing device (10) according to any of the previous claims and a microfluidic system coupled to the nanopore sensing device (10).

**13.** The system according to claim 12, further comprising:

 iii. a cis reservoir (80) and a *trans* reservoir (85); and
 iv. a cis electrode (81) and a *trans* electrode (86), for generating a potential difference for translocating an analyte (90) through the nanopore (60).

**14.** A method for sensing an analyte (90), comprising:

 a. providing an analyte (90) in a nanopore sensing device (10) as defined in any of claims 1 to 13;
 b. translocating the analyte (90) through the nanopore (60); and
 c. sensing an electric feature in the nanopore (60) as the analyte (90) translocates therethrough.

**15.** The method according to claim 14, further comprising:
d. analysing the electric feature sensed in step c to determine therefrom a speed and/or length of the analyte (90) longitudinal to the nanopore (60).

# FIG 1

# FIG 2

**FIG 3**

**FIG 4**

## FIG 5

## FIG 6

Europäisches
Patentamt

European
Patent Office

Office européen
des brevets

# EUROPEAN SEARCH REPORT

Application Number

EP 23 21 9970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 175 195 B2 (BASHIR RASHID [US]; VENKATESAN BALA MURALI [US]; UNIV ILLINOIS [US]) 8 January 2019 (2019-01-08) <br> * page 23, line 19 – line 61 * <br> * page 6, line 26 – line 42 * <br> * figure 21B * | 1-15 | INV. <br> G01N33/487 |
| X | US 10 261 067 B2 (IBM [US]) 16 April 2019 (2019-04-16) <br> * column 8, line 4 – column 9, line 23 * <br> * figures 4A-L * | 1-15 | |
| X | US 2011/279125 A1 (BEDELL STEPHEN W [US] ET AL) 17 November 2011 (2011-11-17) <br> * paragraph [0037] * <br> * figure 15 * | 1-15 | |
| X | US 2015/014752 A1 (D EMIC CHRISTOPHER P [US] ET AL) 15 January 2015 (2015-01-15) <br> * paragraph [0041] – paragraph [0043] * <br> * figure 2 * | 1-15 | |
| A | US 11 367 797 B2 (IMEC VZW [BE]) 21 June 2022 (2022-06-21) <br> * the whole document * | 1-15 | TECHNICAL FIELDS SEARCHED (IPC) <br><br> G01N |
| A | US 2019/145950 A1 (TAKULAPALLI BHARATH [US]) 16 May 2019 (2019-05-16) <br> * paragraph [0054] * | 1-15 | |

—/—

The present search report has been drawn up for all claims

| Place of search | Date of completion of the search | Examiner |
|---|---|---|
| The Hague | 18 April 2024 | Baranski, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 21 9970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | VERHULST ANNE S. ET AL: "Boosting the Sensitivity of the Nanopore Field-Effect Transistor to Translocating Single Molecules", IEEE SENSORS JOURNAL , vol. 22, no. 6 15 March 2022 (2022-03-15), pages 5732-5742, XP093044085, USA ISSN: 1530-437X, DOI: 10.1109/JSEN.2022.3149345 Retrieved from the Internet: URL:https://ieeexplore.ieee.org/iel7/7361/4427201/09705575.pdf * the whole document * | 1-15 | |
| A | US 2012/060589 A1 (GRIDELET EVELYNE [BE] ET AL) 15 March 2012 (2012-03-15) * paragraph [0055] * * figure 1 * | 1-15 | |

TECHNICAL FIELDS
SEARCHED (IPC)

The present search report has been drawn up for all claims

| Place of search | Date of completion of the search | Examiner |
|---|---|---|
| The Hague | 18 April 2024 | Baranski, Jörg |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
...............................................................................................
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

**EP 23 21 9970**

This annex lists the patent family members relating to the patent documents cited in the above-mentioned European search report.
The members are as contained in the European Patent Office EDP file on
The European Patent Office is in no way liable for these particulars which are merely given for the purpose of information.

**18-04-2024**

| Patent document cited in search report | | | Publication date | Patent family member(s) | | | Publication date |
|---|---|---|---|---|---|---|---|
| US 10175195 | B2 | | 08-01-2019 | CN | 104011866 | A | 27-08-2014 |
| | | | | EP | 2737536 | A1 | 04-06-2014 |
| | | | | EP | 3385992 | A1 | 10-10-2018 |
| | | | | JP | 6679106 | B2 | 15-04-2020 |
| | | | | JP | 2014521956 | A | 28-08-2014 |
| | | | | JP | 2017227657 | A | 28-12-2017 |
| | | | | KR | 20140046471 | A | 18-04-2014 |
| | | | | US | 2014174927 | A1 | 26-06-2014 |
| | | | | US | 2019178840 | A1 | 13-06-2019 |
| | | | | WO | 2013016486 | A1 | 31-01-2013 |
| US 10261067 | B2 | | 16-04-2019 | US | 2016313278 | A1 | 27-10-2016 |
| | | | | US | 2018120288 | A1 | 03-05-2018 |
| US 2011279125 | A1 | | 17-11-2011 | CN | 102313769 | A | 11-01-2012 |
| | | | | KR | 20110126545 | A | 23-11-2011 |
| | | | | US | 2011279125 | A1 | 17-11-2011 |
| | | | | US | 2014327446 | A1 | 06-11-2014 |
| US 2015014752 | A1 | | 15-01-2015 | NONE | | | |
| US 11367797 | B2 | | 21-06-2022 | EP | 3502688 | A1 | 26-06-2019 |
| | | | | US | 2021184053 | A1 | 17-06-2021 |
| | | | | WO | 2019120642 | A1 | 27-06-2019 |
| US 2019145950 | A1 | | 16-05-2019 | CN | 109313157 | A | 05-02-2019 |
| | | | | EP | 3446113 | A1 | 27-02-2019 |
| | | | | US | 2019145950 | A1 | 16-05-2019 |
| | | | | WO | 2017184790 | A1 | 26-10-2017 |
| US 2012060589 | A1 | | 15-03-2012 | AT | E535800 | T1 | 15-12-2011 |
| | | | | CN | 102369430 | A | 07-03-2012 |
| | | | | EP | 2237027 | A1 | 06-10-2010 |
| | | | | US | 2012060589 | A1 | 15-03-2012 |
| | | | | WO | 2010113090 | A1 | 07-10-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VERHULST ; ANNE S. et al.** Boosting the Sensitivity of the Nanopore Field-Effect Transistor to Translocating Single Molecules. *IEEE Sensors Journal,* 2022, vol. 22.6, 5732-5742